# EUROPÄISCHE PATENTSCHRIFT

(11) **EP 3 203 950 B1**
(45) Veröffentlichungstag und Bekanntmachung des Hinweises auf die Patenterteilung: **23.12.2020**
(21) Anmeldenummer: 16781054.8
(22) Anmeldetag: 23.08.2016
(51) Int. Cl.: A61F 9/013

(54) **VORRICHTUNG ZUM ANBRINGEN EINER MARKIERUNG AM MENSCHLICHEN AUGE**
DEVICE FOR APPLYING A MARK TO A HUMAN EYE
DISPOSITIF POUR MARQUER UN OEIL HUMAIN

(30) Priorität: 01.09.2015 DE 102015216723
(43) Veröffentlichungstag der Anmeldung: 16.08.2017
(73) Patentinhaber: Geuder AG, 69126 Heidelberg (DE)
(72) Erfinder: EL-AYARI, Hamadi, 60323 Frankfurt (DE); MUELLER-ALBINUS, Julius, 69488 Birkenau (DE); FATH, Hartmut, 69168 Wiesloch (DE)
(74) Vertreter: Patent- und Rechtsanwälte Ullrich & Naumann
(86) Internationale Anmeldenummer: PCT/DE2016/200391
(87) Internationale Veröffentlichungsnummer: WO 2017/036475

(56) Entgegenhaltungen:
- DE-U1-202010 012 367
- US-A- 4 440 168
- US-A1- 2006 229 495
- US-A1- 2009 254 108
- US-A1- 2012 245 609

## Beschreibung

Die Erfindung betrifft eine Vorrichtung zum Anbringen einer Markierung am menschlichen Auge, insbesondere auf der Hornhaut des Auges.

Es besteht die grundsätzliche Notwendigkeit zur Markierung der Hornhaut des menschlichen Auges, nämlich in Vorbereitung ophthalmologischer Eingriffe bei einer Korrektur des Astigmatismus, vorzugsweise zur passgenauen Fixierung einer Linse an der Iris.

Gattungsbildende Vorrichtungen sind bereits aus der Praxis bekannt, meist unter der Bezeichnung "Markierungsinstrument".

Aus DE 20 2008 004 593 U1 ist ein solches Markierungsinstrument bekannt, welches zum Erzeugen von Markierungen für augenchirurgische Eingriffe dient. Das bekannte Instrument umfasst einen Markierungskopf, der schwenkbar mit einem Instrumentengriff verbunden ist. Ein am Instrumentenkopf vorgesehenes Markierungselement ist in seiner Winkellage relativ zu einem Lotgewicht verstellbar angeordnet, so dass Markierungen mit einer Winkeleinstellung relativ zur Lotrechten erzeugbar sind.

US 2009/0 254 108 A1 zeigt ein ähnliches Instrument, nämlich eine Vorrichtung zum Anbringen einer Markierung am menschlichen Auge, genauer gesagt auf der Hornhaut. Das Instrument umfasst einen Markierungskopf und eine den Markierungskopf tragende Halteeinrichtung, wobei der Markierungskopf ein Markierungselement und einen das Markierungselement in einer vorgebbaren bzw. veränderbaren Winkelposition haltbaren Träger aufweist. Der Träger ist drehfest zur Halteeinrichtung oder einer Bezugslinie angeordnet. Der Markierungskopf ist mit der Halteeinrichtung verbunden.

Aus EP 2 453 854 B1 ist eine gattungsbildende Vorrichtung bekannt, wobei dieser Vorrichtung die zuvor genannte US-Druckschrift als Ausgangspunkt zugrunde liegt. Bei der gattungsbildenden Vorrichtung hat der Markierungskopf Kopplungsmittel zum unmittelbaren oder mittelbaren Verbinden mit der Halteinrichtung zum Einstecken eines Anschlussstücks. Das Anschlussstück ist der Halteinrichtung oder einem zwischengeschalteten Adapter zugeordnet. Alternativ kann der Markierungskopf ein Anschlussstück mit einem Einsteckbereich zur unmittelbaren Ankopplung an die Halteeinrichtung umfassen, nämlich zum unmittelbaren Einstecken in einen im Sinne einer geschlitzten Hülse ausgebildeten Konnektor der Halteeinrichtung.

Die gattungsbildende Vorrichtung ist in Bezug auf die Handhabung problematisch, genauer gesagt in Bezug auf das "Finden" des korrekten Markierungsorts. Das Gerät selbst bzw. der Markierungskopf sperrt die Sicht auf die zu markierende Stelle im Auge.

Außerdem ist bei der bekannten Vorrichtung die Lagerung des Markierungskopfes unzureichend. Regelmäßig handelt es sich dort um ein aus Teflon bestehendes Gleitlager, welches einem beachtlichen Verschleiß unterliegt und obendrein bei Verschmutzung nur noch unzureichend seinen Zweck einer einwandfreien Lagerung bei Gewährleistung einer reibarmen Drehbarkeit des Markierungskopfes erfüllt.

Der vorliegenden Erfindung liegt daher die Aufgabe zugrunde, eine Vorrichtung der gattungsbildenden Art derart auszugestalten und weiterzubilden, so dass sie sich mit guter Zielsicherheit einfach handhaben lässt. Die Markierung am Auge soll reproduzierbar sein. Außerdem soll eine einwandfreie und robuste Lagerung des Markierungskopfes gewährleistet sein.

Die voranstehende Aufgabe ist durch die Merkmale des Anspruchs 1 gelöst.

Erfindungsgemäß ist erkannt worden, dass es zur Lagerung des Markierungskopfes oder eines zwischengeschalteten Adapters von Vorteil ist, wenn eine besondere Halteeinrichtung vorgesehen ist. Dazu hat der Markierungskopf bzw. der Adapter einen Lagerbereich, der in einem den Lagerbereich umgreifenden Lagerring der Halteeinrichtung drehbar gelagert ist. Der Lagerring der Halteeinrichtung nimmt somit den Lagerbereich das Markierungskopfes oder des Adapters in sich auf, wobei es von Vorteil ist, wenn der Markierungskopf bzw. der Adapter zur Demontage der Vorrichtung aus dem Lagerring herausnehmbar bzw. herausziehbar ist. Wesentlich ist jedenfalls, dass der Markierungskopf mit seinem Markierungselement drehbar bzw. schwenkbar unmittelbar oder mittelbar in den Lagerring der Halteeinrichtung einsteckbar und darin vorzugsweise frei drehbar ist. Der Lagerring selbst ist zur Handhabung einem Griff, einer Apparatur, einer Einrichtung oder dergleichen zugeordnet, wobei sich der Markierungskopf ungeachtet der Ausrichtung des Griffs, etc. unter Nutzung der Schwerkraft drehen kann. Dies wird später noch erörtert werden.

In vorteilhafter Weise ist zur Lagerung des Markierungskopfes im Lagerring der Halteeinrichtung ein Kugellager vorgesehen, welches zwischen dem Lagerring der Halteeinrichtung und dem Lagerbereich des Markierungskopfes oder des Adapters wirkt. Das Kugellager kann insgesamt aus Edelstahl gefertigt sein. Ebenso ist es denkbar, dass der Käfig des Kugellagers aus Edelstahl und die Kugeln aus verschleißfester Keramik hergestellt sind. Auch das gesamte Lager kann aus Keramik bestehen.

In weiter vorteilhafter Weise weist der Markierungskopf und ggf. der Adapter einen vorzugsweise koaxialen Durchgang auf. Dieser Durchgang endet im Bereich des Markierungselements (distal) mit einem koaxialen Zielröhrchen bzw. einer mittig angeordneten Hülse, durch die hindurch eine Peilung von der Rückseite der Vorrichtung her, durch die Vorrichtung hindurch, möglich ist. Das Zielröhrchen endet vorzugsweise vor dem Wirkbereich des Markierungselements, so dass das Zielröhrchen stets außer Kontakt mit der Hornhaut des Auges bleibt, da zurückversetzt.

Das Markierungselement kann zwei oder vier paarweise einander gegenüberliegender Markierklingen umfassen, wobei die Vorkehrung mindestens zweier Klingen zwingend erforderlich ist. Die Vorkehrung von drei Markierklingen, sternförmig angeordnet, ist ebenfalls denkbar. Bei der Vorkehrung von mehr als zwei Klingen ist ein sicheres, kippfreies Aufsetzen auf der Hornhaut des Auges gewährleistet.

Ungeachtet der Anzahl der Markierklingen ist es möglich, dass diese jeweils zur Markierung einer Geraden oder zumindest zweier Punkte oder Abschnitte einer Geraden ausgebildet sind. Im Konkreten kann die Markierklinge gezackt, vorzugsweise zumindest teilweise der Augenoberfläche angepasst gebogen sein.

In Bezug auf die Lagerung und Bewegung des Markierungselements bzw. des Markierungskopfes ist es von Vorteil, wenn der Markierungskopf in dem Lagerring der Halteeinrichtung, per Hand oder mittels eines Werkzeugs (im weitesten Sinne zu verstehen), drehbar ist, nämlich in eine vorgebbare Winkelposition zur Horizontalen oder Vertikalen.

Auch ist es von Vorteil, wenn zur Anzeige der eingestellten Winkelposition des Markierungselements bzw. der Markierklingen ein Markierungsring vorgesehen ist, der eine die Winkelposition der Halteeinrichtung anzeigende Markierung hat. Des Weiteren ist ein Skalierring des Markierungskopfes vorgesehen, der eine Skalierung zum Anzeigen der Winkelposition durch den Markierring bzw. durch dessen Markierung, vorzugsweise im Bereich von 0° bis 180°, umfasst.

An dieser Stelle sei angemerkt, dass das Markierungselement drehfest mit bzw. in dem Markierungskopf verbunden ist. Der Skalierring des Markierungskopfes, mit seiner Skalierung, ist zu dem Markierungselement bzw. den Markierklingen drehfest ausgebildet. Der Markierungskopf ist drehbar im Lagerring der Halteeinrichtung gelagert. Des Weiteren ist der Markierring der Halteeinrichtung drehbar zum Lagerring angeordnet, jedoch drehfest zum eingesetzten Markierungskopf, so dass dessen die Winkelposition symbolisierende Markierung entsprechend der eingestellten Winkelposition des Markierungskopfes bzw. des Skalierrings auf eine Gradzahl zeigt und die entsprechende Winkelstellung symbolisiert. Die gesamte Anordnung ist im Drehring drehbar, wobei die Winkelstellung des Markierungskopfes vorgebbar ist.

Im Rahmen einer weiteren Ausgestaltung ist es denkbar, dass zwischen dem Markierungskopf und der Halteeinrichtung oder dem Adapter Kopplungsmittel vorgesehen sind, die vorzugsweise Federkraft beaufschlagte Rastmittel zum gegenseitigen Verrasten umfassen. Beliebige Konstruktionen einer geeigneten Kopplung sind denkbar.

Bei der Halteeinrichtung kann es sich um ein herkömmliches ophthalmologisches Gerät handeln, ohne dessen eigentlichen Funktionskopf. Beispielsweise kann eine Spaltlampe dazu dienen.

Bereits zuvor ist erwähnt worden, dass der Markierungskopf unmittelbar oder mittelbar mit dem Gerät, beispielsweise der Spaltlampe, verbunden sein kann. Bei mittelbarer Kopplung kann der Markierungskopf über ein Kopplungsmittel bzw. über einen ein Anschlussstück umfassenden Adapter mit dem Gerät verbunden sein.

Als Halteeinrichtung kann ebenso ein handgehaltenes ophthalmologisches Instrument dienen, vorzugsweise mit einer sich zur Horizontalen oder Vertikalen ausrichtenden, schwerkraftbelasteten Pendelaufnahme für den Markierungskopf. Insoweit sei lediglich beispielhaft auf die EP 2 453 854 B1 verwiesen, die einen sogenannten Pendelmarkierer zeigt.

Bei einem solchen Pendelmarkierer kann das Gewicht und die Länge des den Markierungskopf ausrichtenden Pendels unterschiedlich ausgeführt sein. Ein langes Pendel pendelt länger aus, während ein kurzes Pendel nach nur wenigen Amplituden eingependelt ist. Anstelle eines Pendels können auch Zapfen oder dergleichen vorgesehen sein. Wesentlich ist, dass das Pendel oder der Zapfen eine optische Kontrolle liefert, wobei die Gradzahl der Winkelstellung des Markierungskopfes auf der zuvor erwähnten Skalierung angezeigt wird.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung zweier bevorzugter Ausführungsbeispiele der Erfindung anhand der Zeichnungen zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Ansicht ein erstes Ausführungsbeispiel einer erfindungsgemäßen Vorrichtung in Form eines sogenannten Pendelmarkieres, umfassend einen Markierungskopf mit einem zwei Markierklingen aufweisenden Markierungselement,
- Fig. 2: in einer schematischen Ansicht, vergrößert, den Markierungskopf nebst Halteeinrichtung aus Fig. 1,
- Fig. 3: in einer schematischen Ansicht, von der Rückseite her, die Vorrichtung aus Fig. 1,
- Fig. 4: in einer schematischen Seitenansicht die Vorrichtung aus Fig. 1,
- Fig. 5: in einer schematischen Ansicht, vergrößert, die Vorrichtung aus Fig. 4,
- Fig. 6: in einer schematischen Ansicht die Vorrichtung aus den Fig. 1 bis 5, vergrößert, mit herausgezogenem Markierungskopf,
- Fig. 7: in einer schematischen Ansicht die Vorrichtung aus den Fig. 1 bis 6 von der Rückseite der Halteeinrichtung her gesehen, ohne Markierungskopf,
- Fig. 8: in einer schematischen Seitenansicht den Markierungskopf der Vorrichtung aus den Fig. 1 und 7,
- Fig. 9: in einer schematischen Ansicht, geschnitten, den Gegenstand aus Fig. 8,
- Fig. 10: in einer schematischen Vorderansicht, d.h. von den Markierklingen her gesehen, den Gegenstand aus den Fig. 8 und 9,
- Fig. 11: in einer schematischen Ansicht, vergrößert, den Markierungskopf gemäß den Fig. 6 und 8,
- Fig. 12: in einer schematischen Ansicht ein zweites Ausführungsbeispiel einer erfindungsgemäßen Vorrichtung in Form eines sogenannten Pendelmarkieres, umfassend einen Markierungskopf mit einem vier Markierklingen aufweisenden Markierungselement,
- Fig. 13: in einer schematischen Ansicht, vergrößert, den Markierungskopf nebst Halteeinrichtung aus Fig. 12,
- Fig. 14: in einer schematischen Ansicht, von der Rückseite her, die Vorrichtung aus Fig. 12,
- Fig. 15: in einer schematischen Seitenansicht die Vorrichtung aus Fig. 12,
- Fig. 16: in einer schematischen Ansicht, vergrößert, die Vorrichtung aus Fig. 15,
- Fig. 17: in einer schematischen Ansicht die Vorrichtung aus den Fig. 12 bis 16, vergrößert, mit herausgezogenem Markierungskopf,
- Fig. 18: in einer schematischen Ansicht die Vorrichtung aus den Fig. 12 bis 17 von der Rückseite der Halteeinrichtung her gesehen, ohne Markierungskopf,
- Fig. 19: in einer schematischen Seitenansicht den Markierungskopf der Vorrichtung aus den Fig. 12 und 18,
- Fig. 20: in einer schematischen Ansicht, geschnitten, den Gegenstand aus Fig. 19,
- Fig. 21: in einer schematischen Vorderansicht, d.h. von den Markierklingen her gesehen, den Gegenstand aus den Fig. 19 und 20, und
- Fig. 22: in einer schematischen Ansicht, vergrößert, den Markierungskopf gemäß den Fig. 17 und 19,

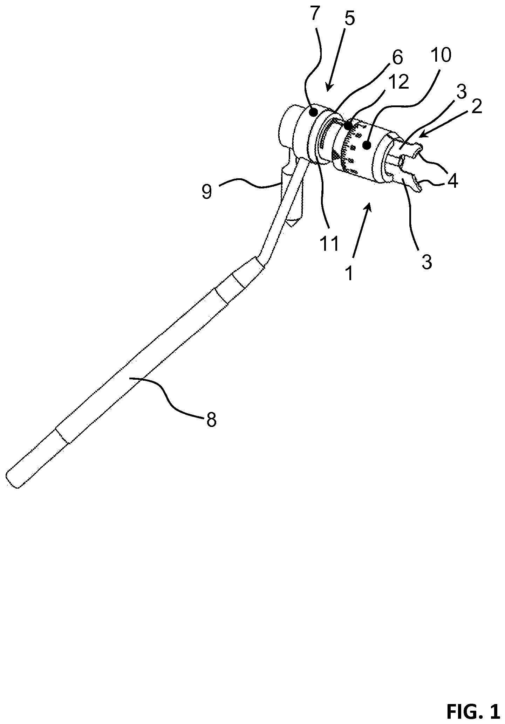

Fig. 1 zeigt ein Ausführungsbeispiel einer erfindungsgemäßen Vorrichtung, wobei es sich hier um einen sogenannten Pendelmarkierer handelt. Der Pendelmarkierer umfasst einen Markierungskopf 1, der ein Markierungselement 2 umfasst. Das Markierungselement 2 hat wiederum zwei Markierklingen 3, die mit gezackten Kontaktflächen 4 ausgestattet sind.

Die Markierklingen 3 bzw. das Markierungselement 2 ist fest in den Markierungskopf 1 eingebunden.

Des Weiteren ist eine Halteeinrichtung 5 vorgesehen, die den Markierungskopf 1 oder einen zwischengeschalteten Adapter trägt. Der Markierungskopf 1 hat einen Lagerbereich 6, der in einem den Lagerbereich 6 umgreifenden Lagerring 7 der Halteeinrichtung 5 in einer vorgebbaren bzw. veränderbaren Winkelposition drehbar gelagert ist.

Der Markierungskopf 1 ist per Hand oder Werkzeug drehbar in den Körper des Lagerbereichs 6 eingesteckt und dort mehr oder weniger drehfest gehalten, wobei die gesamte Anordnung, d.h. der Markierungskopf 1 und der Lagerbereich 6, drehbar im Lagerring 7 angeordnet sind bzw. sich durch diesen hindurch erstrecken.

Der Lagerring 7 ist fest mit einem abgewinkelten Griffteil 8 eines Handinstruments verbunden.

An dem den Markierklingen 3 entgegengesetzten Ende des sich mit dem Lagerbereich 6 durch den Lagerring 7 hindurch erstreckenden Markierungskopfes 1 ist ein Pendel 9 vorgesehen, der den im Lagerring 7 drehbar gelagerten Markierungskopf 1 in seiner durch das Pendel 9 vorgegebenen Position (vertikal bzw. horizontal) hält bzw. ausrichtet. Ganz gleich, wie das Griffteil 8 gehalten wird, richtet das Pendel 9 den Markierungskopf 1 in die voreingestellten Winkelstellung aus.

Zur Einstellung bzw. zur Kontrolle der voreingestellten Winkelstellung des Markierkopfes 1 ist an Markierkopf 1 ein Skalierring 10 als äußerer Ring vorgesehen, der mit einer Skalierung 18 im Bereich von 0° bis 180° ausgestattet ist.

Die den Lagerbereich 6 bildende Hülse 11 weist federgespannte Mittel auf, die den dort eingesteckten Markierungskopf 1 in der jeweiligen Winkelposition halten. Mit der Hülse 11 ist drehfest ein Markierungsring 12 verbunden, der ein Dreieck, einen Pfeil, ein Strich oder dergleichen als Markierung aufweist, die die Winkelposition am Skalierring 10 markiert.

In vorteilhafter Weise lässt sich der Pendelmarkierer gemäß Fig. 1 in seine Bestandteile zerlegen bzw. lassen sich einzelne Teile austauschen.

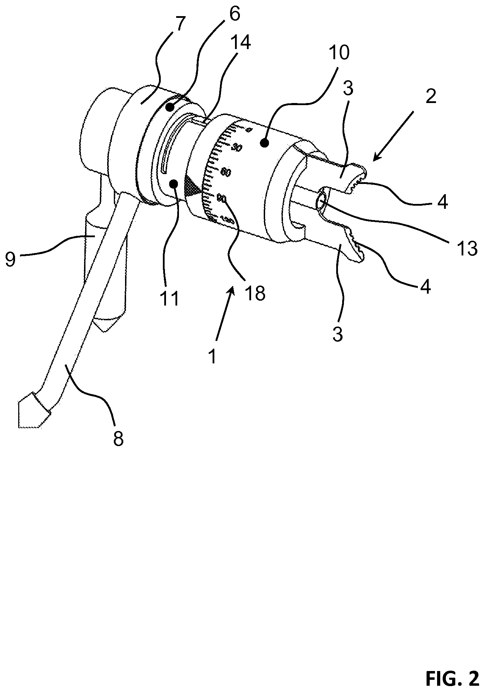

Fig. 2 zeigt den Markierungskopf 1 des Pendelmarkierers aus Fig. 1 vergrößert. Hier ist deutlich erkennbar, dass zwischen den beiden gegenüberliegenden Markierklingen 3 ein Zielröhrchen 13 angeordnet ist, welches sich zumindest um einen gewissen Bereich durch den Markierungskopf 1 hindurch erstreckt. Die gesamte Anordnung weist einen koaxialen Durchgang auf, der auf der dem Markierungselement 2 abgewandten Seite zum Einblick bzw. Durchblick offen ist und zu dem Markierungselement 2 hin in das Zielröhrchen 13 mündet. So lässt sich eine Position am Auge anpeilen, bevor das Markierungselement 2 mit seinen Markierklingen 3 auf die Hornhaut des Auges zu deren Markierung aufgesetzt wird.

Fig. 2 zeigt deutlich die Ausgestaltung des Lagerbereichs 6 bzw. der den Lagerbereich 6 tragenden Hülse 11, die mit Klemmmitteln 14 zum mehr oder weniger drehfesten Festlegen bzw. Festklemmen des Markierungskopfes 1 ausgestattet ist.

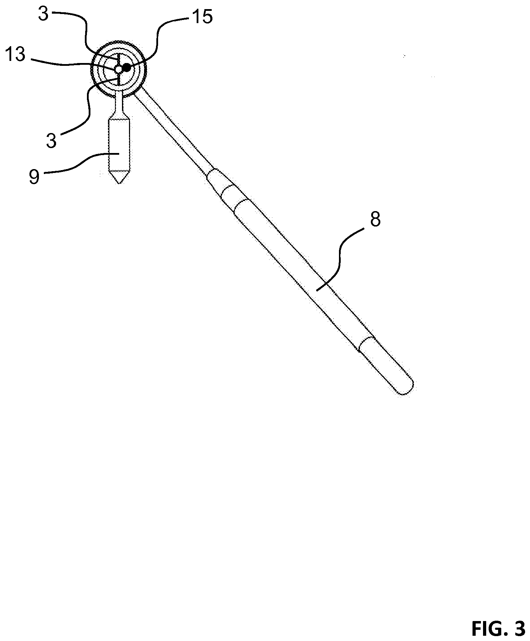

Fig. 3 zeigt den Gegenstand aus Fig. 1 in einer schematischen Ansicht von der Rückseite her, d.h. dem distalen Ende abgewandt. Es lässt sich der koaxiale Durchgang 15 und das mittige Zielröhrchen 13 erkennen. Beidseits des Zielröhrchens 13 sind die Markierklingen 3 angeordnet.

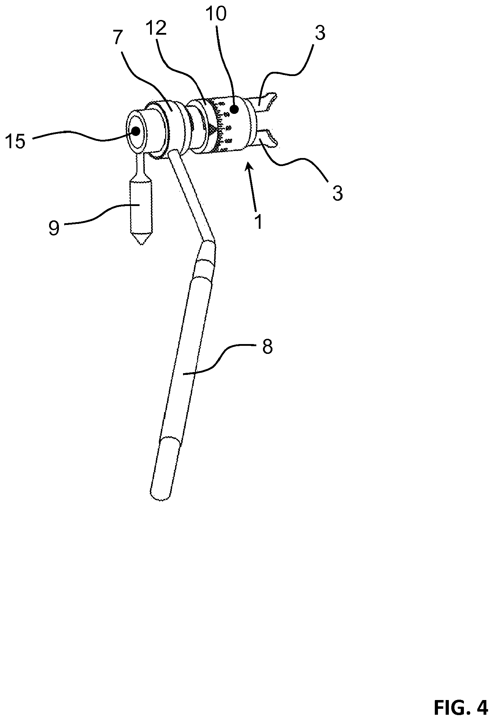

Fig. 4 zeigt den Pendelmarkierer aus Fig. 1 in einer anderen Perspektive. Es ist gut zu erkennen, wie die Markierung des Markierungsrings 12 die Winkelstellung am Skalierring 10 anzeigt, entsprechend der voreingestellten Winkelstellung des Markierungskopfes 1 bzw. des Markierungselements 2 und somit der Markierklingen 3.

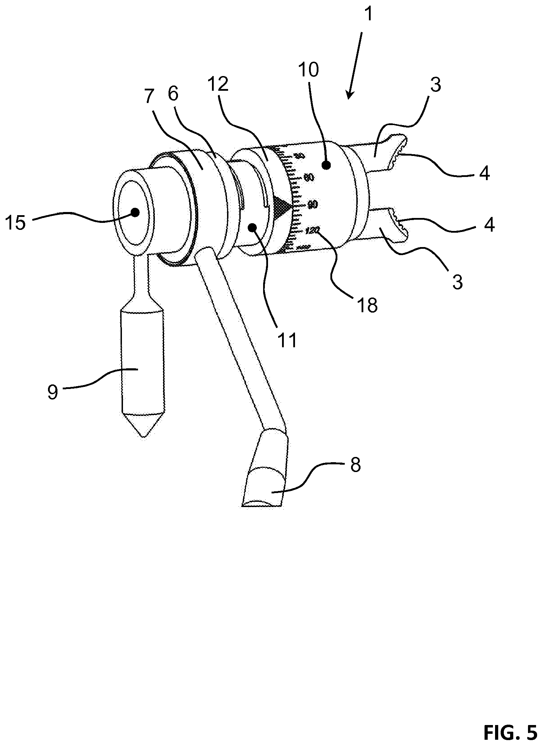

Fig. 5 zeigt den Markierungskopf 1 in vergrößerter Ansicht entsprechend den voranstehenden Ausführungen.

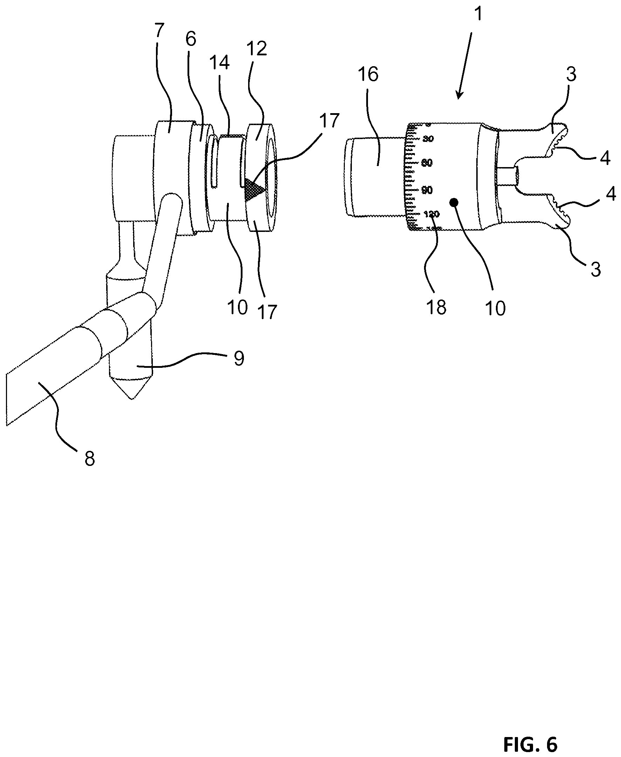

Fig. 6 zeigt den Gegenstand aus Fig. 5, wobei der Markierungskopf 1 mit einer zum Einstecken dienenden Einsteckhülse 16 ausgestattet ist. Diese lässt sich durch den Markierungsring 12 hindurch in die Hülse 11, die den Lagerbereich 6 bildet, unter Überwindung der Klemmkraft der Klemmmittel 14 hineinstecken bzw. hineinpressen. Unter Überwindung der Klemmkraft der Klemmmittel 14 ist die Winkelstellung des Markierungskopfes 1 veränderbar, wobei die Winkelstellung durch die Markierung 17 am Markierungsring 12 gezeigt wird und die Markierung 17 auf die gegenüberliegende Skalierung 18 des Skalierrings 10 zeigt.

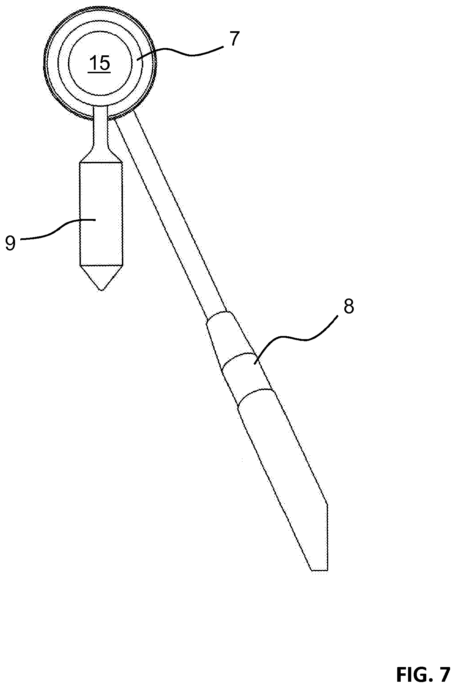

Fig. 7 zeigt den Gegenstand aus Fig. 6 von der Rückseite her, ohne den eingesetzten Markierungskopf. Der koaxiale Durchgang 15 ist komplett frei.

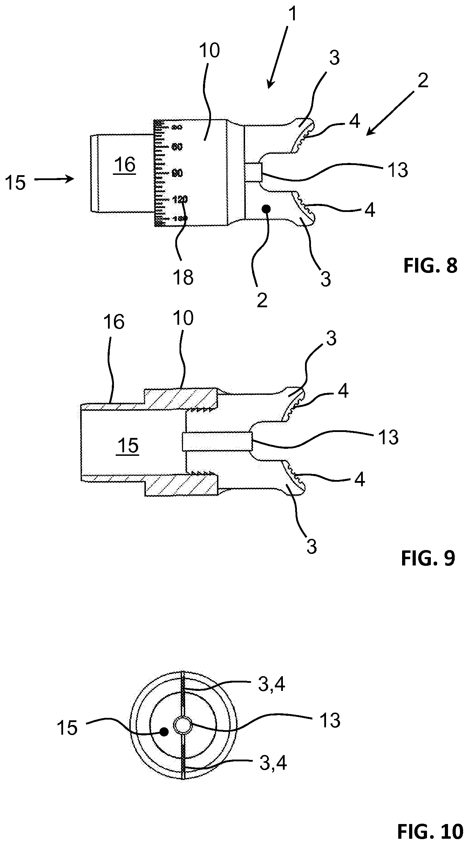

Fig. 8 zeigt für sich gesehen den Markierungskopf 1 mit äußerem Skalierring 10 nebst Skalierung 18. Am distalen Ende befindet sich das Markierungselement 2 mit den beiden Markierklingen 3. Mittig ist das Zielröhrchen 13 angeordnet.

Auf der dem distalen Ende gegenüberliegenden Seite des Markierungskopfes 1 befindet sich die Einsteckhülse 16.

Fig. 9 zeigt den Gegenstand aus Fig. 8, teilweise geschnitten. Die Anordnung und Ausbildung des Zielröhrchens 13 ist erkennbar. Der äußere Skalierring 10 ist einteilig mit der Einsteckhülse 16 ausgeführt.

Fig. 10 zeigt den Gegenstand aus den Fig. 8 und 9 vom distalen Ende her, wobei das koaxiale Zielröhrchen 13 und die Markierklingen 3 nebst den gezahnten Kontaktflächen 4 erkennbar sind.

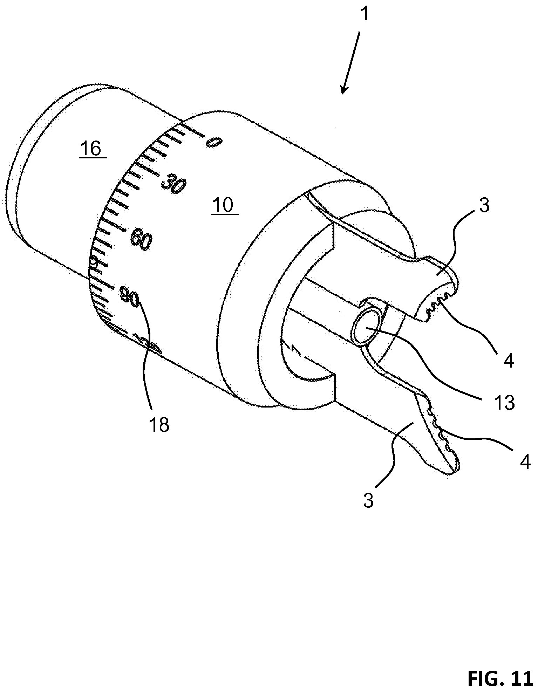

Fig. 11 zeigt in einer vergrößerten Ansicht den Markierungskopf 1, wobei die Vorkehrung des Zielröhrchens 13 besonders gut zu erkennen ist. Der äußere Skalierring 10 ist integral mit der Einsteckhülse 16 ausgebildet, nämlich zum drehfesten Einstecken in die zuvor erörterte Hülse 11, die den Lagerbereich 6 bildet, der im Lagerring 7 drehbar gelagert ist.

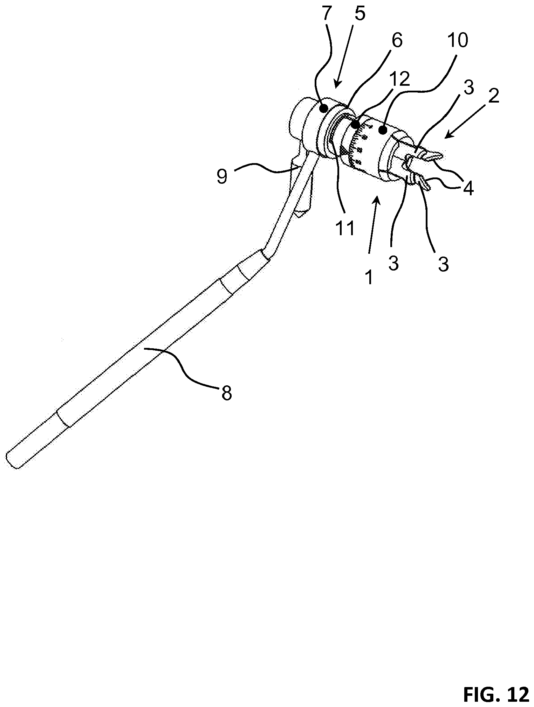

Fig. 12 zeigt ein weiteres Ausführungsbeispiel des Pendelmarkierers aus den Fig. 1 bis 11, welche sich gegenüber dem Ausführungsbeispiel aus den Fig. 1 bis 11 dadurch unterscheidet, dass das Markierungselement 2 insgesamt vier paarweise gegenüberliegende Markierklingen 3 aufweist. Das mittige Zielröhrchen gemäß Ausführungsbeispiel aus den Fig. 1 bis 11 ist dort nicht vorhanden, wobei ein rückseitiger Durchblick durch die gesamte Anordnung möglich ist. Ansonsten weist dieses Ausführungsbeispiel die gleichen Merkmale wie das Ausführungsbeispiel aus den Fig. 1 bis 11 auf, so dass weitere detaillierte Ausführungen dazu nicht erforderlich sind.

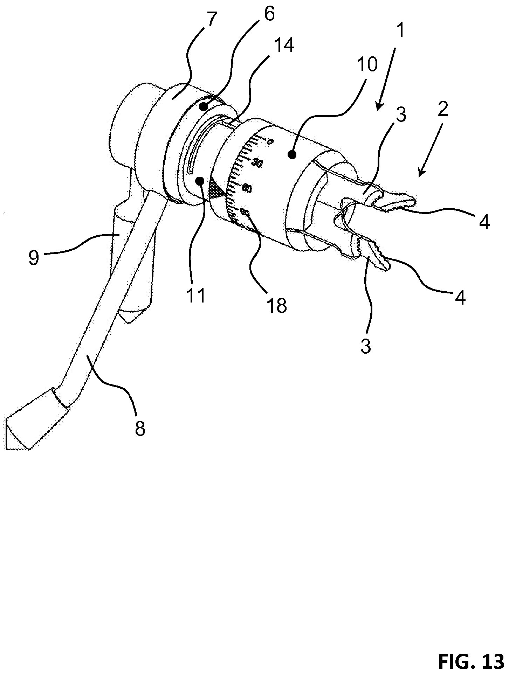

Fig. 13 zeigt das Ausführungsbeispiel aus Fig. 12 mit vergrößertem Markierungskopf 1. Das Markierungselement 2 mit insgesamt vier Markierklingen 3 ist dort besonders gut zu erkennen.

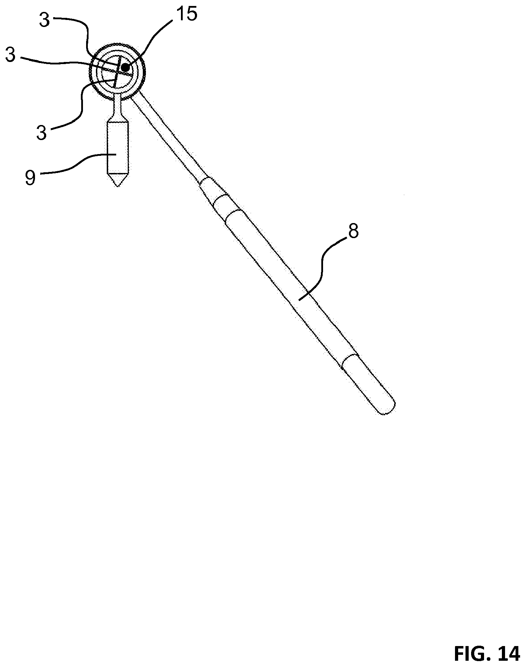

Fig. 14 zeigt den Gegenstand aus den Fig. 12 und 13 von der Rückseite her, wobei diese Ansicht den koaxialen Durchgang 15 erkennen lässt. Am distalen Ende ist das Markierungselement 2 mit den vier Markierklingen 3 angeordnet.

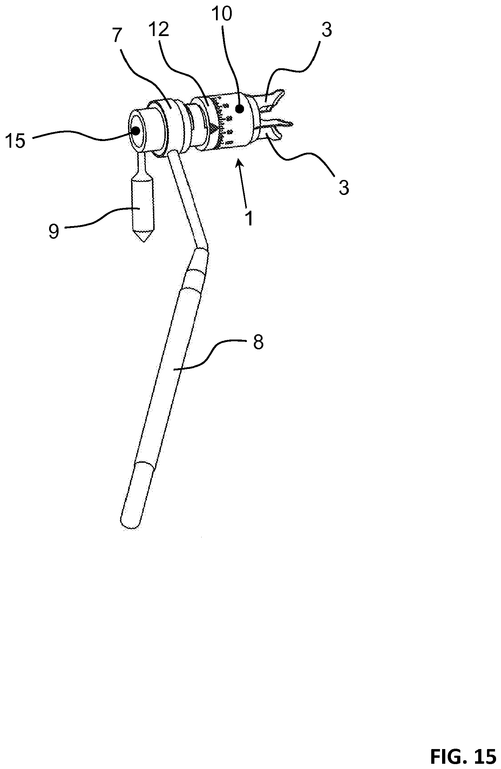

Fig. 15 zeigt den Gegenstand aus den Fig. 12 bis 14 unter einer anderen Perspektive, wo auch dort das Markierungselement 2 mit den vier Markierklingen 3 erkennbar ist.

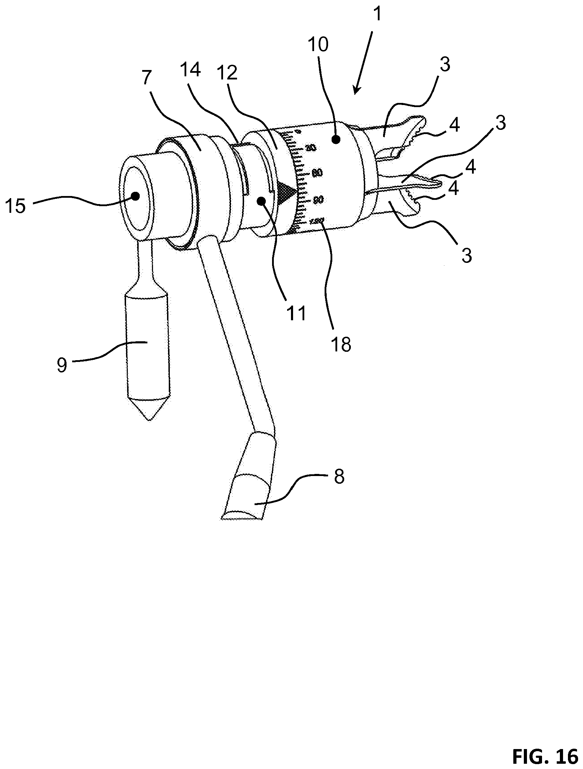

Fig. 16 zeigt den Gegenstand aus Fig. 15 in einer vergrößerten Ansicht, im Detail das Markierungselement 2 mit den vier Markierklingen 3.

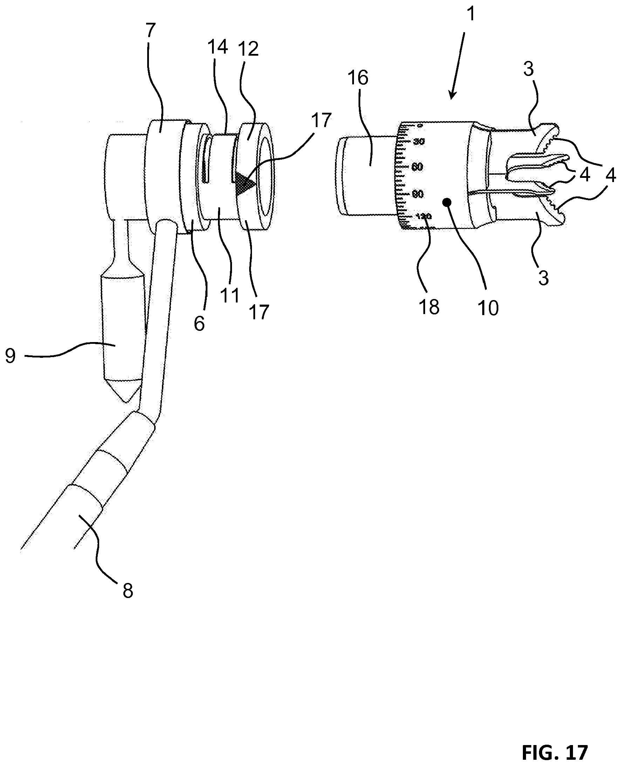

Fig. 17 zeigt das Ausführungsbeispiel der Fig. 12 bis 16 mit herausgezogenem Markierungskopf 1. Die Einsteckhülse 16 ist deutlich zu erkennen, die durch den Markierungsring 12 hindurch in die Hülse 11 des Lagerbereichs 6 unter Überwindung der Klemmkraft von Klemmmitteln 14 steckbar und in einer Winkelstellung festlegbar ist. Zur Einstellung und zum Ablesen der Winkelstellung sei zur Vermeidung von Wiederholungen auf die Ausführungen zum ersten Ausführungsbeispiel verwiesen.

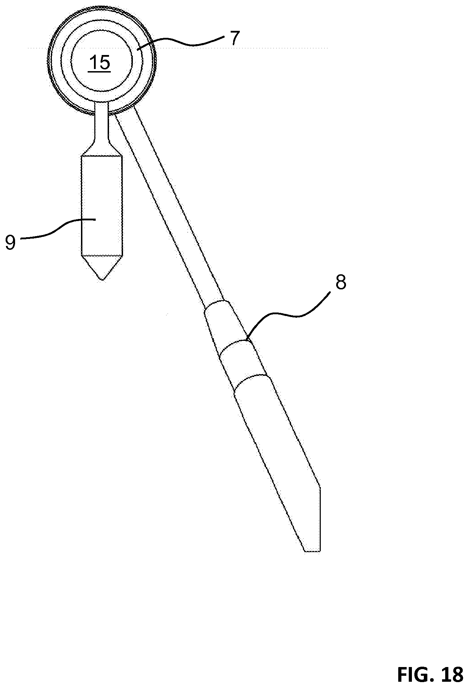

Fig. 18 zeigt den Gegenstand aus Fig. 17 ohne Markierungskopf. Der koaxiale Durchgang 15 ist frei.

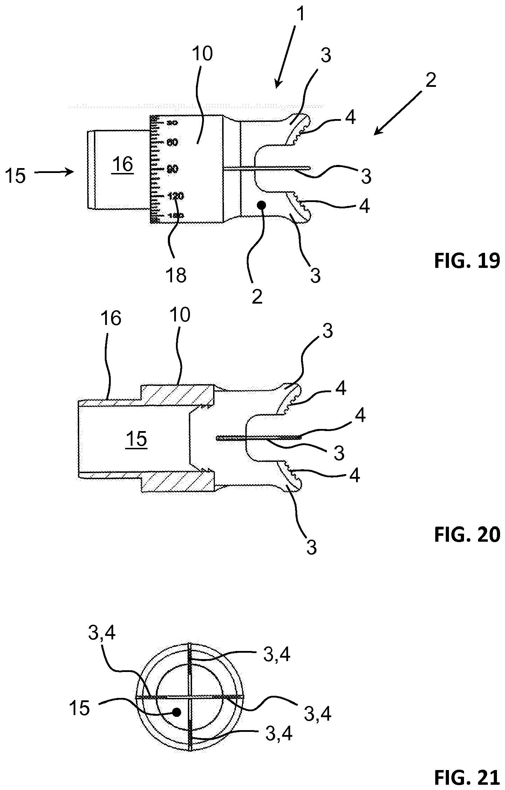

Fig. 19 zeigt den Markierungskopf 1 des Ausführungsbeispiels aus den Fig. 12 bis 18, mit einem Markierungselement 2, welches insgesamt vier Markierklingen 3 umfasst.

Fig. 20 zeigt den Gegenstand aus Fig. 19 teilweise geschnitten. Auch hier sind die vier Markierklingen 3 deutlich zu sehen. Der Skalierring 10 und die Einsteckhülse 16 sind integrale Bestandteile des Markierungskopfes 1.

Fig. 21 zeigt von distalen Ende her den Gegenstand aus den Fig. 19 und 20, nämlich den Markierungskopf 1. Es ist klar erkennbar, dass das Markierungselement 2 insgesamt vier Markierklingen 3 umfasst.

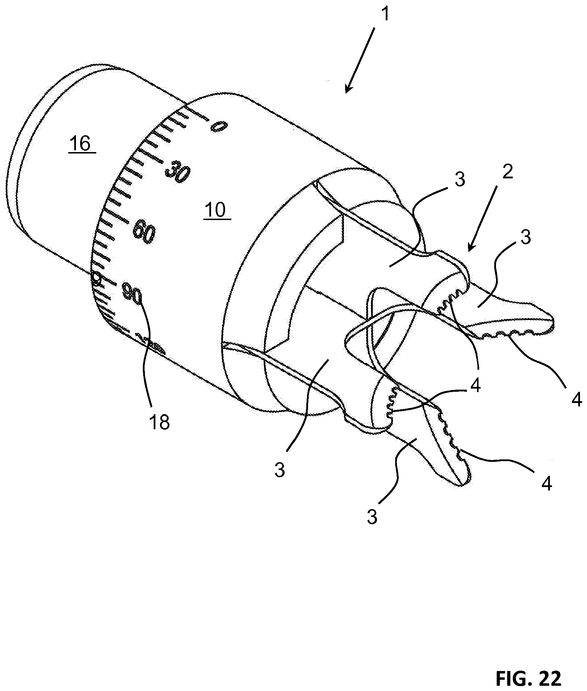

Schließlich zeigt Fig. 22 den Markierungskopf 1 des Ausführungsbeispiels der Fig. 1 bis 21 in vergrößerter Ansicht. Das Markierungselement 2 hat vier Markierklingen 3, jeweils am distalen Ende mit Kontaktflächen 4, die gezackt sind. Der Begriff "Kontaktfläche" ist im weitesten Sinne zu verstehen. Es kann sich dabei auch um eine Kontaktlinie bzw. aufgrund der gezackten Ausführung um Kontaktpunkte handeln.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken.

### Bezugszeichenliste

- 1: Markierungskopf
- 2: Markierungselement
- 3: Markierklinge
- 4: Kontaktfläche, Kontaktlinie, Kontaktpunkt (der Markierklinge)
- 5: Halteeinrichtung
- 6: Lagerbereich
- 7: Lagerring
- 8: Griffteil
- 9: Pendel, Gewicht
- 10: Skalierring
- 11: Hülse (bildet den Lagerbereich)
- 12: Markierungsring
- 13: Zielröhrchen
- 14: Klemmmittel
- 15: koaxialer Durchgang
- 16: Einsteckhülse
- 17: Markierung (auf dem Markierungsring)
- 18: Skalierung

## Patentansprüche

1. Vorrichtung zum Anbringen einer Markierung am menschlichen Auge, insbesondere auf der Hornhaut des Auges,
mit einem ein Markierungselement (2) umfassenden Markierungskopf (1) und einer den Markierungskopf (1) oder einen zwischengeschalteten Adapter tragenden Halteeinrichtung (5),
wobei der Markierungskopf (1) oder der Adapter einen Lagerbereich (6) aufweist,
**dadurch gekennzeichnet, dass** der Markierungskopf (1) drehbar in den Körper des Lagerbereichs (6) eingesteckt und dort drehfest gehalten ist, und
dass der Lagerbereich (6) in einem den Lagerbereich (6) umgreifenden Lagerring (7) der Halteeinrichtung (5) in einer vorgebbaren bzw. veränderbaren Winkelposition, in dieser eingestellt, drehbar gelagert ist.

2. Vorrichtung nach Anspruch 1, **dadurch gekennzeichnet, dass** zur Lagerung des Lagerbereichs (6) des Markierungskopfes (1) im Lagerring (7) der Halteeinrichtung (5) (oder umgekehrt) ein Kugellager vorgesehen ist.

3. Vorrichtung nach Anspruch 2, **dadurch gekennzeichnet, dass** das Kugellager aus Edelstahl und/oder aus Keramik besteht.

4. Vorrichtung nach einem der Ansprüche 1 bis 3, **dadurch gekennzeichnet, dass** der Markierungskopf (1) und ggf. der Adapter einen vorzugsweise koaxialen Durchgang aufweist, der im Bereich des Markierungselements (2) mit einem koaxialen Zielröhrchen (13) endet.

5. Vorrichtung nach einem der Ansprüche 1 bis 4, **dadurch gekennzeichnet, dass** das Markierungselement (2) zwei oder vier paarweise einander gegenüberliegende Markierklingen (3) umfasst.

6. Vorrichtung nach Anspruch 5, **dadurch gekennzeichnet, dass** die Markierklingen (3) vorzugsweise zur Markierung einer Geraden oder mindestens zweier Punkte oder Abschnitte einer Geraden ausgebildet sind.

7. Vorrichtung nach Anspruch 5 oder 6, **dadurch gekennzeichnet, dass** die Markierklingen (3) gezackt, vorzugsweise zumindest teilweise der Augenoberfläche angepasst gebogen sind.

8. Vorrichtung nach einem der Ansprüche 1 bis 7, **dadurch gekennzeichnet, dass** der Markierungskopf (1) per Hand oder mittels eines Werkzeugs in seiner Winkelstellung einstellbar bzw. veränderbar ist.

9. Vorrichtung nach einem der Ansprüche 1 bis 8, **dadurch gekennzeichnet, dass** ein Markierungsring (12) der Halteeinrichtung (5) eine die Winkelposition symbolisierende Markierung und ein Skalierring (10) des Markierungskopfes (1) eine Skalierung (18) zum Anzeigen der Winkelposition, vorzugsweise im Bereich von 0° bis 180°, umfasst.

10. Vorrichtung nach einem der Ansprüche 1 bis 9, **dadurch gekennzeichnet, dass** zwischen dem Markierungskopf (1) und der Halteeinrichtung (5) oder dem Adapter wirkende Kopplungsmittel vorgesehen sind, die vorzugsweise federkraftbeaufschlagte Rastmittel zum gegenseitigen Festlegen oder Verrasten umfassen.

11. Vorrichtung nach einem der Ansprüche 1 bis 10, **dadurch gekennzeichnet, dass** als Halteeinrichtung (5) ein herkömmliches ophthalmologisches Gerät, ohne dessen eigentlichen Funktionskopf, beispielsweise eine Spaltlampe, dient.

12. Vorrichtung nach Anspruch 11, **dadurch gekennzeichnet, dass** der Markierungskopf (1) über ein Kopplungsmittel bzw. einen ein Anschlussstück umfassenden Adapter mit dem Gerät verbunden ist.

13. Vorrichtung nach einem der Ansprüche 1 bis 10, **dadurch gekennzeichnet, dass** als Halteeinrichtung (5) ein handgehaltenes ophthalmologisches Instrument, vorzugsweise mit einer sich zur Horizontalen oder Vertikalen ausrichtenden, schwerkraftbelasteten Pendelaufnahme für den Markierungskopf (1), dient.

## Claims

1. Device for applying a mark to the human eye, in particular to the cornea of the eye,
having a marking head (1) which comprises a marking element (2) and a retention device (5) which carries the marking head (1) or an adapter which is arranged therebetween,
wherein the marking head (1) or the adapter has a bearing region (6),
**characterised in that** the marking head (1) is rotatably inserted in the body of the bearing region (6) and is retained at that location in a rotationally secure manner, and
**in that** the bearing region (6) is rotatably supported in a bearing ring (7) of the retention device (5) which engages round the bearing region (6) in a predeterminable or variable angular position, in a state adjusted therein.

2. Device according to claim 1, **characterised in that** a ball bearing is provided in order to support the bearing region (6) of the marking head (1) in the bearing ring (7) of the retention device (5) (or vice versa).

3. Device according to claim 2, **characterised in that** the ball bearing comprises high-grade steel and/or ceramic material.

4. Device according to any one of claims 1 to 3, **characterised in that** the marking head (1) and where applicable the adapter has/have a preferably coaxial passage which terminates in the region of the marking element (2) with a coaxial sighting tube (13).

5. Device according to any one of claims 1 to 4, **characterised in that** the marking element (2) comprises two or four marking blades (3) which are arranged in pairs opposite each other.

6. Device according to claim 5, **characterised in that** the marking blades (3) are preferably constructed for marking a straight line or at least two points or portions of a straight line.

7. Device according to claim 5 or 6, **characterised in that** the marking blades (3) are jagged, preferably bent so as to be at least partially adapted to the eye surface.

8. Device according to any one of claims 1 to 7, **characterised in that** the marking head (1) can be adjusted or changed in terms of its angular position by hand or by means of a tool.

9. Device according to any one of claims 1 to 8, **characterised in that** a marking ring (12) of the retention device (5) has a mark which indicates the angular position and a scaling ring (10) of the marking head (1) comprises a scale (18) in order to indicate the angular position, preferably in the range from 0° to 180°.

10. Device according to any one of claims 1 to 9, **characterised in that** there are provided coupling means which are active between the marking head (1) and the retention device (5) or the adapter and which preferably comprise locking means which are acted on with resilient force for mutual securing or engagement.

11. Device according to any one of claims 1 to 10, **characterised in that** a conventional ophthalmological device, without the actual functional head thereof, for example, a slit lamp, is used as a retention device (5).

12. Device according to claim 11, **characterised in that** the marking head (1) is connected to the device by means of an adapter which comprises a coupling means or a connecting piece.

13. Device according to any one of claims 1 to 10, **characterised in that** a hand-held ophthalmological instrument, preferably having a gravity-loaded oscillating receiving member which is orientated relative to the horizontal or vertical for the marking head (1), is used as a retention device (5).

## Revendications

1. Dispositif d'application d'un marquage sur l'œil humain, en particulier sur la cornée de l'œil, avec
une tête de marquage (1) comprenant un élément de marquage (2) et avec un dispositif de maintien (5) portant la tête de marquage (1) ou un adaptateur intercalé,
la tête de marquage (1) ou l'adaptateur comportant une zone de palier (6), **caractérisé en ce que** la tête de marquage (1) est enfichée de façon rotative dans le corps de la zone de palier (6) et y est maintenue bloquée en rotation, et
**en ce que**, dans une bague de palier (7) du dispositif de maintien (5) enserrant la zone de palier (6), la zone de palier (6) est supportée de façon rotative dans une position angulaire pré-définissable ou respectivement variable, de façon réglée dans celle-ci.

2. Dispositif selon la revendication 1, **caractérisé en ce qu'**il est prévu un roulement à billes pour supporter la zone de palier (6) de la tête de marquage (1) dans la bague de palier (7) du dispositif de maintien (5) (ou inversement).

3. Dispositif selon la revendication 2, **caractérisé en ce que** le roulement à billes est composé d'acier fin et/ou de céramique.

4. Dispositif selon l'une des revendications 1 à 3, **caractérisé en ce que** la tête de marquage (1) et éventuellement l'adaptateur comportent un passage de préférence coaxial qui se termine dans l'élément de marquage (2) par un petit tube de visée (13) coaxial.

5. Dispositif selon l'une des revendications 1 à 4, **caractérisé en ce que** l'élément de marquage (2) comprend deux ou quatre lames de marquage (3) opposées les unes aux autres par paires.

6. Dispositif selon la revendication 5, **caractérisé en ce que** les lames de marquage (3) sont constituées de préférence pour le marquage d'une droite ou d'au moins deux points ou segments d'une droite.

7. Dispositif selon la revendication 5 ou 6, **caractérisé en ce que** les lames de marquage (3) sont dentelées, de préférence courbées de façon au moins partiellement adaptée à la surface de l'œil.

8. Dispositif selon l'une des revendications 1 à 7, **caractérisé en ce que** la position angulaire de la tête de marquage (1) peut être réglée ou respectivement modifiée manuellement ou au moyen d'un outil.

9. Dispositif selon l'une des revendications 1 à 8, **caractérisé en ce qu'**une bague de marquage (12) du dispositif de maintien (5) comprend un marquage symbolisant la position angulaire, et une bague graduée (10) de la tête de marquage (1) comprend une graduation (18) destinée à indiquer la position angulaire, de préférence dans la plage de 0° à 180°.

10. Dispositif selon l'une des revendications 1 à 9, **caractérisé en ce qu'**il est prévu des moyens d'accouplement agissant entre la tête de marquage (1) et le dispositif de maintien (5) ou l'adaptateur, qui comprennent des moyens d'encliquetage de préférence exposés à une force de ressort pour l'immobilisation ou l'encliquetage réciproque.

11. Dispositif selon l'une des revendications 1 à 10, **caractérisé en ce qu'**un appareil ophtalmologique classique, sans sa tête fonctionnelle proprement dite, par exemple une lampe à fente, sert de dispositif de maintien (5).

12. Dispositif selon la revendication 11, **caractérisé en ce que** la tête de marquage (1) est raccordée à l'appareil par le biais d'un moyen d'accouplement ou respectivement d'un adaptateur comprenant une pièce de raccordement.

13. Dispositif selon l'une des revendications 1 à 10, **caractérisé en ce qu'**un instrument ophtalmologique porté à la main, de préférence avec une monture pendulaire pour la tête de marquage (1), chargée par la force de la gravité et qui s'oriente par rapport à l'horizontale ou à la verticale, sert de dispositif de maintien (5).
